(19) **Europäisches Patentamt / European Patent Office / Office européen des brevets**

(11) **EP 4 602 206 B1**

(12) **EUROPÄISCHE PATENTSCHRIFT**

(45) Veröffentlichungstag und Bekanntmachung des Hinweises auf die Patenterteilung:
**21.01.2026 Patentblatt 2026/04**

(21) Anmeldenummer: **23793240.5**

(22) Anmeldetag: **13.10.2023**

(51) Internationale Patentklassifikation (IPC):
*D06M 16/00* (2006.01)  *C11D 3/386* (2006.01)
*D06P 5/13* (2006.01)  *D06L 4/40* (2017.01)
*D06P 5/15* (2006.01)  *D06M 15/09* (2006.01)
*D06M 101/06* (2006.01)

(52) Gemeinsame Patentklassifikation (CPC):
D06M 16/003; C11D 3/0036; C11D 3/222;
C11D 3/382; C11D 3/38645; C12N 9/2437;
C12N 9/2445; D06L 4/40; D06M 15/01;
D06M 15/05; D06M 15/3562; D06M 15/507;
D06M 15/53; D06M 23/08; D06P 5/137;  (Forts.)

(86) Internationale Anmeldenummer:
**PCT/EP2023/078449**

(87) Internationale Veröffentlichungsnummer:
**WO 2024/079306 (18.04.2024 Gazette 2024/16)**

(54) **BIOSTONING**

BIOSTONING

LAVAGE BIOSTONING

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **14.10.2022 DE 102022127017**
**26.06.2023 DE 102023116704**

(43) Veröffentlichungstag der Anmeldung:
**20.08.2025 Patentblatt 2025/34**

(73) Patentinhaber: **Rudolf GmbH**
**82538 Geretsried (DE)**

(72) Erfinder: **HAUSER, Christoph**
**82538 Geretsried (DE)**

(74) Vertreter: **Weickmann & Weickmann PartmbB**
**Richard-Strauss-Strasse 80**
**81679 München (DE)**

(56) Entgegenhaltungen:
**DE-A1- 102005 049 908**

(52) Gemeinsame Patentklassifikation (CPC): (Forts.)
**D06P 5/158; D06P 7/00;** D06M 15/09;
D06M 2101/06

**Beschreibung**

Die Erfindung betrifft eine Zusammensetzung zum Aufhellen von gefärbten cellulosehaltigen textilen Flächengebilden, um einen "Used"-Effekt zu erzielen.

Die "Used-Optik" ist bei Jeans ein gängiges Fashion-Element und wird durch sogenannte "Stone-washing"-Verfahren erreicht. "Stone-washing" leitet sich von den Bimssteinen ab, die üblicherweise zur Behandlung der Textilien in der Waschflotte eingesetzt werden. Dabei wird an der Oberfläche der Jeans kontrolliert das Textil aufgeraut und dadurch oberflächlich Farbstoff entfernt. Durch das "Stone-washing" erhält die Jeans den erwünschten "Used-Look", d.h. die Jeans ist an manchen Stellen verblasst und wirkt getragen. Probleme beim "Stone-washing" bereiten häufig das unerwünschte Ablagern des Farbstoffabriebs auf dem Textil (sogenanntes "Backstaining") und die Bildung von Faserknötchen an der Oberfläche des Textils (sogenanntes "Pilling"). "Pilling" entsteht durch lose Faserenden, die sich z. B. nach einer Abrasionsbehandlung aus dem Textil lösen und durch mechanische Reibung später Faserknötchen entwickeln.

Die Behandlung mit Bimsstein birgt Nachteile. In den Taschen der Jeans können sich Teile der Steine ablagern. Diese müssen anschließend mühsam von Hand entfernt werden. Zudem können Teile der Bimssteine zu Ablagerungen in Leitungen und Abflüssen führen. Darüber hinaus sind die Waschmaschinen durch die Verwendung von Bimsseinen einem höheren Verschleiß ausgesetzt. Des Weiteren besteht auch die Gefahr, dass die Jeans durch die Bimssteine zu stark geschädigt wird.

Um die Nachteile von Bimsstein zu minimieren, wurde vorgeschlagen, den ausschließlich mechanischen Prozess zum Teil um eine enzymatische Behandlung zu ergänzen.

Aus dem Stand der Technik ist bekannt, dass Cellulasen auf enzymatischem Weg in der Lage sind, einen "Stonewashed"-Effekt zu erzielen. In der Offenlegungsschrift WO 90/07569 A1 wird die Applikation einer sauren Cellulase zusammen mit Bimssteinen vorgestellt.

Aufgrund der Nachteile von Bimsstein wird in der Offenlegungsschrift DE 196 43 036 A1 vorgeschlagen, den Bimsstein komplett durch Siliciumdioxid zu ersetzen. Das vorgeschlagene Waschverfahren kann zudem eine Cellulase enthalten. Weitere abrasive Materialien als Substitut für Bimssteine werden in den Offenlegungsschriften EP 2 142 698 A1 (Silikonpolymer und Aluminosilikat) und CN 1762910 A (keramische Materialien bestehend aus Magnesiumtonen, Kaolin, Feldspat und Dolomit) vorgeschlagen.

Nachteilig bei diesen Verfahren ist die Applikation in einer Flotte, wodurch große Mengen an Abwasser entstehen. Aus Umweltschutzaspekten schlägt die Offenlegungsschrift WO 2022/106072 A1 deshalb ein Verfahren vor, bei dem eine feste Formulierung, bestehend aus einer neutralen Cellulase, einem Füllstoff, einem nicht-ionischen Tensid, einem Anti-Backstaining-Mittel, einem Dispergiermittel und einem pH-Regulator, verwendet wird. Der Füllstoff wird bevorzugt ausgewählt aus Tektosilikat, Calciumcarbonat, Calcium-Magnesiumcarbonat, Kaolin, Glimmer, Talkum, Titandioxid, Wollastonit und/oder Mischungen daraus. Die feste Formulierung wird ohne zusätzliches Wasser auf die feuchte Jeans appliziert und bei Raumtemperatur behandelt.

Auch wenn die genannten Abrasiva Vorteile gegenüber Bimsstein aufweisen, sind vor allem Silicumdioxid (Silica) und Silikate (z. B. Kaolin) als kritisch zu sehen, weil sie lungengängiges kristallines Silica enthalten, was zu Silikose führen kann. Bei den beschriebenen Alternativen handelt es sich auch um natürliche nicht nachwachsende Mineralien, die der Natur entnommen werden müssen und damit zur Verknappung der Ressourcen beitragen. Außerdem können die harten Materialien (die Mohs-Härte für Silikat beträgt etwa 7) die Waschmaschinen genau wie Bimsstein verschleißen und zu Ablagerungen in Leitungen führen.

Das Dokument DE 10 2005 049908 A1 betrifft die Verwendung von Cholinoxidase in verschiedenen Produkten, einschließlich Produkten zur Behandlung, insbesondere zum Bleichen, von Textilien. Es offenbart auch Enzyme wie Cellulase, zum Beispiel Glucanase, um Effekte wie Stone-Washing zu erzielen.

Im Hinblick auf die beschriebenen Nachteile besteht daher die Aufgabe, eine verbesserte Zusammensetzung bereitzustellen, die nicht nur zu guten "Stone-washed" Ergebnissen führt, sondern auch die Nachteile der bisher bekannten "Stone-wash" Prozesse überwindet, also insbesondere gesundheitlich unbedenklich, biologisch abbaubar und nachhaltig ist.

Die Erfindung betrifft daher eine Zusammensetzung, insbesondere eine trockene Zusammensetzung zur Behandlung von textilen Flächengebilden, enthaltend

(a) mindestens eine Cellulase,
(b) mindestens ein ligninhaltiges Material,
(c) mindestens einen pH-Regulator,
(d) gegebenenfalls mindestens ein Tensid und
(e) gegebenenfalls mindestens ein Anti-Backstaining-Mittel.

Die erfindungsgemäße Zusammensetzung kann als Pulver, Granulat, Pellet oder einer Mischung davon vorliegen.

Überraschenderweise wurde gefunden, dass die erfindungsgemäße Zusammensetzung im Vergleich zum Stand der Technik einen verbesserten "Stone-washed"-Effekt bei geringerem Backstaining und vergleichbarem Pilling bewirkt.

Diese Verbesserungen sind insbesondere überraschend, da der Fachmann davon ausgehen musste, dass die Cellulasen in Gegenwart von ligninhaltigen Materialien gebunden und dadurch inhibiert werden (Pan Xj, Role of functional groups in lignin inhibition of enzymatic hydrolysis of cellulose to glucose, J. Biobased Mater. Bioenergy 2008, 2: 25-32). Zumindest musste eine Kompetition von cellulosischem Substrat und dem ligninhaltigen Material vermutet werden, was den "Stone-washed"-Effekt vermindert hätte.

Das ligninhaltige Material ist gesundheitlich und ökologisch unbedenklich und stammt aus biologisch-abbaubaren Abfallprodukten, so dass keine Ressourcen verschwendet werden.

Bevorzugt werden mit der erfindungsgemäßen Zusammensetzung, insbesondere trockenen Zusammensetzung, textile Flächengebilde behandelt, ausgewählt aus natürlichen cellulosischen Fasern, wie Baumwolle, Leinen, Hanf, Jute und Ramie, künstlichen cellulosischen Fasern, wie Viskose, Celluloseacetat, Cellulosetriacetat, Rayon, Cupro, Modal, Lyocell sowie Mischungen davon ggf. mit anderen synthetischen Fasern.

Es wird insbesondere eine neutrale Cellulase verwendet, d.h. eine Cellulase, die ein Aktivitätsmaximum im neutralen Bereich (insbesondere bei einem pH von 6,5-7,5) aufweist. Geeignete Cellulasen können Endo-1,4-$\beta$-Glucanasen, Exo-1,4-$\beta$-Glucanasen, $\beta$-Glucosidasen und Mischungen davon, noch stärker bevorzugt Endo-1,4-$\beta$-Glucanasen sein. Bevorzugt ist die Enzymaktiviät der eingesetzten Cellulase(n) mindestens 300 U/g, stärker bevorzugt 1.000-10.000 U/g und am meisten bevorzugt 5.000-9.000 U/g. In einer besonders bevorzugten Ausführungsform wird eine Cellulase gemäß CAS Nummer 9012-54-8 verwendet.

Die Cellulase oder Komponente a) kann in Pulverform oder als Granulat oder Pellet, bevorzugt als Granulat vorliegen.

In einer bevorzugten Ausführungsform beträgt der Anteil an Komponente (a) 0,01-5 Gew.-%, stärker bevorzugt 0,05-2 Gew.-% und am meisten bevorzugt 0,1-1 Gew.-% bezogen auf die trockene Gesamtzusammensetzung.

Entsprechend beträgt der Anteil an Komponente (a) 0,01-4 Gew.-%, stärker bevorzugt 0,04-1,6 Gew.-% und am meisten bevorzugt 0,1-0,8 Gew.-% bezogen auf die Gesamtzusammensetzung.

Das ligninhaltige Material (b) stammt bevorzugt aus Biomasse. Das ligninhaltige Material der Komponente (b) ist bevorzugt abrasiv und/oder scharfkantig. Das ligninhaltige Material (b) zeichnet sich insbesondere durch einen hohen Ligninanteil aus. In einer bevorzugten Ausführungsform weist das ligninhaltige Material (b) 16-45 Gew.-%, stärker bevorzugt 25-45 Gew.-%, noch stärker bevorzugt 30-45 Gew.-% Lignin bezogen auf die Gesamtmasse der Komponente (b) auf.

In einer bevorzugten Ausführungsform enthält das ligninhaltige Material (b) 30-45 Gew.-% Cellulose, stärker bevorzugt 34-43 Gew.-%, Cellulose bezogen auf die Gesamtmasse der Komponente (b).

Schließlich weist das ligninhaltige Material (b) bevorzugt 20-30 Gew.-%, stärker bevorzugt 22-29 Gew.-%, Hemicellulose bezogen auf die Gesamtmasse der Komponente (b) auf. In einer besonders bevorzugten Ausführungsform enthält das ligninhaltige Material (b):

> 16-45 Gew.-%, stärker bevorzugt 25-45 Gew.-%, Lignin,
> 30-45 Gew.-%, stärker bevorzugt 34-43 Gew.-%, Cellulose und
> 20-30 Gew.-%, stärker bevorzugt 22-29 Gew.-%, Hemicellulose,
> jeweils bezogen auf die Gesamtmasse der Komponente (b).

In einer bevorzugten Ausführungsform ist das ligninhaltige Material der Komponente (b) keine natürliche oder synthetische Faser, die einen Ligninanteil von $\leq$ 5 Gew.-% bezogen auf die Gesamtmasse der Faser aufweist. In einer Ausführungsform ist das ligninhaltige Material (b) verschieden von dem textilen Flächbengebilde. Insbesondere enthält das ligninhaltige Material der Komponente (b) nicht natürliche cellulosische Fasern, wie Baumwolle, Leinen, Hanf, Jute und Ramie, künstliche cellulosische Fasern, wie Viskose, Celluloseacetat, Cellulosetriacetat, Rayon, Cupro, Modal, Lyocell sowie Mischungen davon. Insbesondere ist das ligninhaltige Material (b) auch keine Waschnuss.

Der Anteil der Komponente (b) liegt bevorzugt im Bereich von 5-99 Gew.-%, stärker bevorzugt 10-20 Gew.-%, bezogen auf die Gesamtmasse der trockenen Zusammensetzung.

Der Anteil der Komponente (b) liegt bevorzugt im Bereich von 4-80 Gew.-%, stärker bevorzugt 8-30 Gew.-%, bezogen auf die Gesamtmasse der Zusammensetzung.

Das ligninhaltige Material der Komponente (b) zeichnet sich bevorzugt durch einen durchschnittlichen Partikeldurchmesser von 100-250 $\mu$m, stärker bevorzugt von 120-200 $\mu$m und am stärksten bevorzugt von 140-170 $\mu$m aus. Partikel, die einen größeren oder kleineren durchschnittlichen Durchmesser besitzen, weisen einen wesentlich geringeren "Stone-washed"-Effekt auf.

Der mittlere Partikeldurchmesser wird gemäß DIN 66165 gemessen.

Die geeignete Partikelgröße des ligninhaltigen Materials kann durch konventionelle Mahlmethoden und ggf. anschließendes Sieben gewonnen werden. Geeignete Mahlverfahren sind beispielsweise das Behandeln mit einer Schlag-, Hammerschlag-, Stift-, Feinprallmühle ggf. mit Pendel- oder Plattenschlägerwerk, Konduxmühle, oder einem Passagenwalzwerk.

Das ligninhaltige Material (b) hat bevorzugt eine Mohshärte im Bereich von 1,5-5,5, stärker bevorzugt von 2-4 gemessen gemäß DIN EN 101.

Die Materialien weisen damit eine geeignete Härte auf, die einerseits die abrasive Wirkung des Materials sicherstellt, andererseits die Maschinenteile beim "Stone-washing" nicht signifikant angreift.

In einer besonders bevorzugten Ausführungsform wird das ligninhaltige Material (b) aus Nussschalen und/oder Kernschalen gewonnen.

In einer besonders bevorzugten Ausführungsform wird das ligninhaltige Material (b) aus Nussschalen gewonnen. Geeignete Nussschalen sind Walnuss-, Haselnuss-, Mandel-, Kokosnuss-, Pistazien-, Macadamianuss-, Pekannussschalen oder Mischungen davon. Kernschalen sind bevorzugt Kernschalen aus Steinobst, insbesondere Oliven, Aprikosen, Pfirsiche, Kirschen und Pflaumen, Pinien, Palmfrüchte, Jojobafrüchte, Kakaobohnen oder Mischungen davon.

Das ligninhaltige Material (b) enthält bevorzugt keine Samen oder Nüsse. Die Bestandteile der Samen und Nüsse, insbesondere die Fette, beeinflussen das "Stone-washing" nachteilig.

Das ligninhaltige Material (b) enthält bevorzugt maximal 3 Gew.-%, stärker bevorzugt 0,01-3 Gew.-%, noch stärker bevorzugt 1-2 Gew.-%, Petrolether-lösliche Bestandteile bezogen auf die Gesamtmasse der Komponente (b). Die Masse der Petroletherlöslichen Bestandteile wird in Anlehnung an ISO 734 bestimmt.

Die Kern- und/oder Nussschalen enthalten bevorzugt weitere Inhaltsstoffe. Entsprechend weisen die ligninhaltigen Materialien (b) ferner Mono- und/oder Polysaccharide, stärker bevorzugt Saccharose, Glukose, Xylose, Mannitol und/oder Myoinositol auf. Ohne an eine Theorie gebunden zu sein, können die Mono- oder Polysaccharide, insbesondere Saccharose, Glukose, Xylose, Mannitol und Myoinositol als Aktivatoren der Cellulase wirken. Entsprechend eignen sich die abrasiven ligninhaltigen Materialien der Komponente (b) insbesondere für die erfindungsgemäße Zusammensetzung.

Das ligninhaltige Material (b) hat bevorzugt eine Schüttdichte von 300-700 kg/m$^3$, stärker bevorzugt 400-600 kg/m$^3$. Die Schüttdichte wird in Anlehnung an DIN ISO EN 60 bestimmt.

Die erfindungsgemäße Zusammensetzung enthält mindestens einen pH-Regulator. Der pH-Regulator gewährleistet die Einstellung des pH-Werts, bei dem die Cellulase ihre höchste Aktivität aufweist. Geeignete pH-Regulatoren sind im Fachgebiet bekannt und ausgewählt aus einem wasserlöslichen anorganischen und/oder organischen Salz, stärker bevorzugt einem Phosphat-, Sulfat-, Carbonat-, Citrat- oder Acetatsalz. Bevorzugt ist der pH-Regulator (c) ausgewählt aus dem Alkalimetallsalz der Ortho-, Di- oder Triphosphorsäure, der Schwefelsäure, der Kohlensäure, der Zitronensäure und/oder der Essigsäure. Bevorzugt ist der pH-Regulator (c) ausgewählt aus dem Alkalimetallsalz der Ortho-, Di- oder Triphosphorsäure, der Schwefelsäure, der Kohlensäure, der Zitronensäure, der Essigsäure, der Adipinsäure und/oder Tris(hydroxymethyl)aminomethan- (TRIS-) Puffer, 3-(N-Morpholino)propansulfon-säure- (MOPS-) Puffer und/oder 2-(4-(2-Hydroxyethyl)-1-piperazinyl)-ethansulfonsäure- (HEPES-) Puffer.

Bevorzugt ist Komponente (c) eine Mischung aus Alkalisalzen der Ortho-, Di- oder Triphosphorsäure mit Alkalisalzen der Schwefelsäure, insbesondere eine Mischung aus Natriumphosphat und Natriumsulfat.

Der pH-Regulator der Komponente (c) kann im Bereich von 1-80 Gew.-%, stärker bevorzugt 10-70 Gew.-% und am stärksten bevorzugt 40-70 Gew.-% bezogen auf die Gesamtmasse der trockenen Zusammensetzung ausmachen.

Der pH-Regulator der Komponente (c) kann im Bereich von 1-60 Gew.-%, stärker bevorzugt 10-40 Gew.-% und am stärksten bevorzugt 30-40 Gew.-% bezogen auf die Gesamtmasse der Zusammensetzung ausmachen.

Entsprechend dem Aktivitätsmaximum der neutralen Cellulase hat die erfindungsgemäße Zusammensetzung bevorzugt einen pH-Wert von 5-8, stärker bevorzugt einen pH-Wert von 6,5-7,5, gemessen als 1 %-ige wässrige Lösung der Zusammensetzung bei Raumtemperatur.

Die Zusammensetzung kann ferner mindestens ein Tensid (d) enthalten. Das Tensid ist bevorzugt ein nicht-ionisches Tensid und stärker bevorzugt ein polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid. In einer bevorzugten Ausführungsform ist Komponente (d) ausgewählt aus der Gruppe bestehend aus alkoxylierten $C_9$-$C_{25}$-Fettalkoholen, alkoxylierten $C_9$-$_{25}$-Fettsäureaminen, alkoxylierten $C_9$-$C_{25}$-Fettsäureamiden, an der Carboxylatfunktion alkoxylierten $C_8$-$C_{25}$-Fettsäuren, alkoxylierten $C_8$-$C_{25}$-Fettsäureestern, alkoxylierten $C_8$-$C_{25}$-Alkylphenolen und alkoxylierten Mono-, Di- oder Triglyceriden von $C_8$-$C_{25}$-Fettsäuren und/oder deren Veresterungsprodukten mit $C_8$-$C_{25}$-Fettsäuren oder Trialkylphenylpolyalkoxylen oder einem Block-Polymer, z.B. Poly(ethylenoxid-co-propylenoxid), oder Fettalkohol-poly(ethylenoxid-co-propylenoxid) sowie Mischungen davon. Die Anzahl der Alkoxylengruppen im nicht-ionischen Tensid beträgt mindestens 4, bevorzugt 8-100, weiter bevorzugt 10-85 und am meisten bevorzugt 20-80 Wiederholungseinheiten. Die Alkylgruppen können jeweils unabhängig voneinander verzweigt oder geradkettig, gesättigt oder ungesättigt sein.

Bevorzugte nichtionische Tenside (d) sind alkoxylierte $C_{12}$-$C_{18}$-Fettalkohole mit 50-100 Wiederholungseinheiten der Alkoxylengruppen.

Geeignete nichtionische Tenside besitzen bevorzugt einen HLB-Wert von 9 bis 11, 11 bis 14 oder > 14, insbesondere > 14.

Der HLB-Wert bezeichnet den hydrophilen und lipophilen Anteil der nicht-ionischen Tenside (W. C. Griffin: Classification of surface active agents by HLB. In: J. Soc. Cosmet. Chem. 1, 1949, S. 311-326).

Die Komponente (d) unterstützt gleichzeitig die Dispergierung der ligninhaltigen Materialien und die Benetzung des zu behandelnden textilen Flächengebildes mit den Komponenten der Zusammensetzung.

Das Tensid der Komponente (d) kann 0-40 Gew.-%, bevorzugt 5-30 Gew.-% am meisten bevorzugt 10-20 Gew.-%, bezogen auf die Gesamtmasse der trockenen Zusammensetzung ausmachen.

Das Tensid der Komponente (d) kann 0-30 Gew.-%, bevorzugt 4-20 Gew.-% am meisten bevorzugt 10-15 Gew.-%, bezogen auf die Gesamtmasse der Zusammensetzung ausmachen.

Beim "Stone-washing" werden absichtlich Farbstoffpartikel von den textilen Flächengebilden entfernt. Um zu verhindern, dass sich die Farbstoffpartikel an anderen Stellen ablagern, insbesondere an Stellen, an denen eine Ablagerung überhaupt nicht erwünscht ist, also an nicht oder anders gefärbten Stellen, insbesondere Innenhosentaschen und Labels, werden üblicherweise Anti-Backstaining-Mittel eingesetzt. Bekannte Anti-Backstaining-Mittel der Komponente (e) sind PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol oder polymeres Polycarboxylat. Die Anti-Backstaining-Mittel dienen als "Chelatoren" der Farbstoffpartikel und emulgieren und entfernen die Farbstoffpartikel, die vom Stoff abgerieben und in der Spülflüssigkeit suspendiert werden.

Das Anti-Backstaining-Mittel (e) ist bevorzugt mit 0-10 Gew.-%, bevorzugt 1-5 Gew.-% und stärker bevorzugt 2-3 Gew.-% bezogen auf die Gesamtmasse der trockenen Zusammensetzung enthalten.

Das Anti-Backstaining-Mittel (e) ist bevorzugt mit 0-8 Gew.-%, stärker bevorzugt 2-3 Gew.-%, bezogen auf die Gesamtmasse der Zusammensetzung enthalten.

Insbesondere hat die erfindungsgemäße Zusammensetzung einen Wassergehalt von ≤ 25 Gew.-%, bevorzugt ≤ 10 Gew.-% und am meisten bevorzugt von 0,1-5 Gew.-%, bezogen auf die Gesamtmasse der Zusammensetzung. Der Wassergehalt kann gravimetrisch oder durch Karl-Fischer-Titration bestimmt werden.

Alternativ ist die erfindungsgemäße Zusammensetzung bevorzugt trocken. Insbesondere hat die erfindungsgemäße Zusammensetzung einen Wassergehalt von < 5 Gew.-%, stärker bevorzugt von 0,001-2 Gew.-%, bezogen auf die Gesamtmasse der trockenen Zusammensetzung. Der Wassergehalt kann gravimetrisch oder durch Karl-Fischer-Titration bestimmt werden.

Die erfindungsgemäße Zusammensetzung hat bevorzugt eine Schüttdichte von 800-1.500 kg/m$^3$, stärker bevorzugt von 1.000-1.200 kg/m$^3$.

In einem weiteren Aspekt betrifft die Erfindung einen Kit umfassend mindestens zwei getrennt voneinander vorliegende Einheiten, wobei

die erste Einheit umfasst:

(a) mindestens eine Cellulase, insbesondere ausgewählt aus Endo-1,4-β-Glucanasen, Exo-1,4-β-Glucanasen und β-Glucosidasen,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und
(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat; und

die zweite Einheit umfasst:

(b) mindestens ein ligninhaltiges Material, das bevorzugt 16-45 Gew.-% Lignin bezogen auf die Gesamtmasse der Komponente (b) enthält,
(c) mindestens einen pH-Regulator, insbesondere ausgewählt aus einem wasserlöslichen anorganischen Salz und/oder wasserlöslichen organischen Salz,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und

(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat;

oder

die erste Einheit umfasst:

(a) mindestens eine Cellulase, insbesondere ausgewählt aus Endo-1,4-β-Glucanasen, Exo-1,4-β-Glucanasen und β-Glucosidasen,

(c) mindestens einen pH-Regulator, insbesondere ausgewählt aus einem wasserlöslichen anorganischen Salz und/oder wasserlöslichen organischen Salz,

(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und

(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat; und

die zweite Einheit umfasst:

(b) mindestens ein ligninhaltiges Material, das bevorzugt 16-45 Gew.-% Lignin bezogen auf die Gesamtmasse der Komponente (b) enthält,

(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und

(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat.

Der Kit enthält die Komponenten (a)-(e) bevorzugt in trockener Form, d.h. mit einem Wassergehalt von < 5 Gew.-%, stärker bevorzugt von 0,001-2 Gew.-%, bezogen auf die Gesamtmasse der Komponenten.

Die Komponenten (a)-(e) des Kits entsprechen den jeweiligen Komponenten der erfindungsgemäßen (trockenen) Zusammensetzung. Die vereinigten Komponenten der ersten Einheit und der zweiten Einheit ergeben die erfindungsgemäße Zusammensetzung. Die oben genannten Merkmale der erfindungsgemäßen Zusammensetzung gelten entsprechend für die Gesamtheit der jeweiligen Komponenten in der ersten und zweiten Einheit des Kits.

In einer Ausführungsform der vorliegenden Erfindung kann die erfindungsgemäße Zusammensetzung oder der Kit - wie oben beschrieben - zur Behandlung von textilen Flächengebilden (siehe oben), insbesondere zum Aufhellen von gefärbten textilen Flächengebilden verwendet werden.

Die erfindungsgemäße Zusammensetzung oder der Kit eignet sich hervorragend in "Stone-washing" Anwendungen, um den textilen Flächengebilden und Textilien einen "Used-Look" zu verleihen. Überraschenderweise ist auch der Backstaining-Effekt gegenüber herkömmlichen Zubereitungen beim "Stone-washing" weniger ausgeprägt. Ohne an eine Theorie gebunden zu sein, wird angenommen, dass die erfindungsgemäße Kombination aus Cellulase (a) und dem ligninhaltigen Material (b) synergistisch miteinander wirken.

In einem weiteren Aspekt betrifft die vorliegende Erfindung ein Verfahren zur Behandlung von textilen Flächengebilden, umfassend die Schritte

(i) Bereitstellen eines wasserfeuchten textilen Flächengebildes,

(ii) Zugeben der erfindungsgemäßen Zusammensetzung oder des erfindungsgemäßen Kits,

(iii) Bewegen der nach Schritt (ii) erhaltenen Mischung in einem Behälter,

(iv) Waschen des nach Schritt (iii) erhaltenen textilen Flächengebildes und

(v) Isolieren und ggf. Trocknen des nach Schritt (iv) erhaltenen textilen Flächengebildes.

Es hat sich überraschenderweise gezeigt, dass die erfindungsgemäße Zusammensetzung im trockenen und/oder rieselfähigen Zustand auf das feuchte textile Flächengebilde aufgetragen werden kann, ohne dass hierzu eine wässrige Flotte gebildet werden muss.

Im Falle eines Kits wird im Schritt (ii) entweder die erste Einheit vor der zweiten Einheit oder die zweite Einheit vor der ersten Einheit auf das feuchte textile Flächengebilde aufgetragen.

Bevorzugt hat das textile Flächengebilde im Schritt (i) einen Wassergehalt von 70-100 Gew.-% Wasser, stärker bevorzugt 90-100 Gew.-%, bezogen auf das Trockengewicht des textilen Flächengebildes. Der Wassergehalt wird gemäß DIN 54201 gemessen.

In einer alternativen Ausführungsform hat das textile Flächengebilde im Schritt (i) einen Wassergehalt von 70-400 Gew.-% Wasser, stärker bevorzugt 200-300 Gew.-% Wasser, bezogen auf das Trockengewicht des textilen Flächengebildes. Der Wassergehalt wird gemäß DIN 54201 gemessen.

Bevorzugt ist das textile Flächengebilde gefärbt. Übliche Farbstoffe sind Schwefel-, Reaktiv-, Direkt-, VAT-, Pigment-, natürliche Farbstoffe oder Mischungen davon, besonders bevorzugt sind mit Pigmentfarbstoffen gefärbte textile Flächengebilde, insbesondere indigogefärbte textile Flächengebilde.

Das Gewichtsverhältnis von trockenem textilen Flächengebilde zu erfindungsgemäßer Zusammensetzung in Schritt (ii) ist bevorzugt 100 : 0,5-10, stärker bevorzugt 100:1-10, stärker bevorzugt 100:0,5-5, stärker bevorzugt 100:1-5, stärker bevorzugt 100 : 2-5.

Schritt (iii) erfolgt bevorzugt in einer Waschtrommel. Übliche Waschtrommeln sind dem Fachmann bekannt und können in Haushaltswaschmaschinen, Industriewaschmaschinen, etc. verbaut sein. Schritt (iii) wird bevorzugt bei 20-60, stärker bevorzugt 20-40 U/min durchgeführt. Typischerweise wird Schritt (iii) 10-60 Minuten, bevorzugt 35-45 Minuten durchgeführt. Insbesondere wird Schritt (iii) bei Raumtemperatur (20-25 °C) durchgeführt. Höhere Temperaturen bis 60 °C (wie z.B. 15-60 °C oder 20-30 °C) können eingestellt werden. Alternativ wird Schritt (iii) insbesondere bei 15-25 °C durchgeführt. Höhere Temperaturen bis 60 °C (wie z.B. 20-60 °C oder 30-40°C) können eingestellt werden. Die Deaktivierungstemperatur der Cellulase ist bei der Einstellung der Temperatur zu beachten.

Nach Schritt (iii) wird das erhaltene textile Flächengebilde gewaschen. Bevorzugt erfolgt Schritt (iv) direkt in dem in Schritt (iii) verwendeten Behälter. Schritt (iv) erfolgt üblicherweise durch Zugabe von Wasser (Leitungswasser oder destilliertes Wasser), dem ggf. handelsübliche Waschmittel, Weichspüler, Tenside, etc. zugesetzt sein können.

Der Waschprozess in Schritt (iv) wird bevorzugt so oft wiederholt, bis sich der Anteil an Komponente (b) um mindestens 90 Prozentpunkte, stärker bevorzugt 95 Prozentpunkte, gegenüber dem Anteil der Komponente (b) im Schritt (ii) reduziert hat.

Der Waschprozess wird z.B. 1-5 mal bei einem Flottenverhältnis von 1:5 - 1:7 wiederholt, d.h. bei einer Jeans mit einem Trockengewicht von 556 g werden 2780 g - 3892 g Wasser, insbesondere Leitungswasser, verwendet.

Im Schritt (v) wird das nach Schritt (iv) erhaltene textile Flächengebilde üblicherweise zunächst geschleudert und ggf. getrocknet.

Geschleudert wird bevorzugt 2-10 min, stärker bevorzugt 3-5 min, bei bevorzugt 100-1000 Umdrehungen pro Minute, stärker bevorzugt 300- 500 Umdrehungen pro Minute.

Das Trocknen erfolgt in handelsüblichen Industrie- oder Haushaltstrocknern. Dem Fachmann sind entsprechende Trockner wie Kondensations-, Ventilations- oder Wärmepumpentrockner sowie Spannrahmen bekannt. Das Trocknen erfolgt bevorzugt bei 20-90 °C, stärker bevorzugt bei 30-75 °C.

Die vorliegende Erfindung wird anhand der folgenden Beispiele erläutert:

**Beipiele**

Beispiel 1 (Vergleich mit Silikat-Abrasivum)

Es wurde eine erfindungsgemäße Zubereitung A mit einer silikathaltigen Zusammensetzung B des Standes der Technik verglichen. Die jeweiligen Zusammensetzungen und ihre Eigenschaften können der Tabelle 1 entnommen werden.

Ein Paar fabrikneuer Denim-Jeans (indigo- und schwefelgefärbtes Baumwollgewebe) mit einem Gewicht von ca. 556 g wurden in eine Trommelwaschmaschine des Typs TONELLO G1 Modell 70 L1 Ecofree (Fassungsvermögen 70 l) mit 1670 g Wasser versetzt und überschüssiges Wasser entfernt (abgepumpt).

Zu den feuchten Jeans wurden anschließend je 55 g der Zusammensetzung A und B in trockener Form zugesetzt und bei 20°C für 30 min bei 24 Umin$^{-1}$ mit Umkehr der Rotationsrichtung alle zwei Minuten behandelt.

Tabelle 1: Zusammensetzungen A -G

| Bestandteile | Zusammensetzung A | Zusammensetzung B (Vergleich) | Zusammensetzung C (Vergleich) | Zusammensetzung D (Vergleich) | Zusammensetzung E (Vergleich) | Zusammensetzung F | Zusammensetzung G |
|---|---|---|---|---|---|---|---|
| | Gew.-% | Gew.-% | Gew.-% | Gew.-% | Gew.-% | Gew.-% | Gew.-% |
| Mandelkernschalenmehl, durchschnittlicher Partikeldurchmesser: 160 $\mu$m | 10 | - | - | 10 | - | - | 16 |
| Mandelkernschalenmehl, durchschnittlicher Partikeldurchmesser: 350 $\mu$m | | - | - | - | - | 10 | - |
| Silikat Durchs.Partikeldurchmesser: 8 $\mu$m | - | 10 | - | - | - | - | - |
| Phosphatsalz (c) | 40 | 40 | 40 | 40 | 40 | 40 | 40 |
| $C_{14}$-$C_{18}$-Fettalkoholethoxylat, 80 EO (d) | 13 | 13 | 13 | 13 | 13 | 13 | 13 |
| Sulfatsalz (c) | 23 | 23 | 23 | 23 | 23 | 23 | 23 |
| neutrale Cellulase-Zubereitung (a) (0,3 g Enzym mit 8000 U/g) | 12 | 12 | - | - | 12 | 12 | 6 |
| nicht-ionisches Polyesterpolymer (e) | 2 | 2 | 2 | 2 | 2 | 2 | 2 |
| Schüttdichte gemäß DIN EN ISO 60 [kg / m$^3$] | 1080 | 1076 | | | | | |

Die Jeans wurden anschließend für 1 min bei 20°C in jeweils 2780 g Leitungswasser gespült. Das Wasser wurde in einer Schleuder der Marke TONELLO Modell Hydro 12 5 min bei 300 Umin$^{-1}$ und anschließend in einem Trockner "TONELLO G1 Modell TD 25 E" bei 70°C ca. 40 min vollständig entfernt.

Es wurde die Änderung der Helligkeit am jeweils rechten Hosenbein außen (Abrasiv-Effekt) und an der jeweils rechten Innentasche untersucht (Backstaining-Effekt). Außerdem wurde das Gewebe visuell auf Pilling geprüft.

Die Ergebnisse der Jeans A (Zusammensetzung A) und Jeans B (Zusammensetzung B) sind in Tabelle 2 zusammengefasst.

Tabelle 2: Ergebnisse der "Stone-Washing"-Behandlung

| Ergebnis | Jeans A | Jeans B (Vergleich) |
|---|---|---|
| Farbintensität [%] | 96,9 | 100 |
| Backstaining an der Hoseninnentasche Farbintensität [%] | 84,5 | 100 |
| Backstaining an der Hoseninnentasche [DL] | -0,5 | 0 |

"DL" bezeichnet die Änderung der Helligkeit bzw. Dunkelheit.

"Farbintensität" und "Farbtiefe" bezeichnen jeweils die Farbstärke und werden synonym verwendet.

Bei der Farbmessung gemäß Beispiel 1 wurde die Jeans, die mit einer Zusammensetzung gemäß dem Stand der Technik behandelt wurde, als Standard mit 100 % Farbtiefe gesetzt.

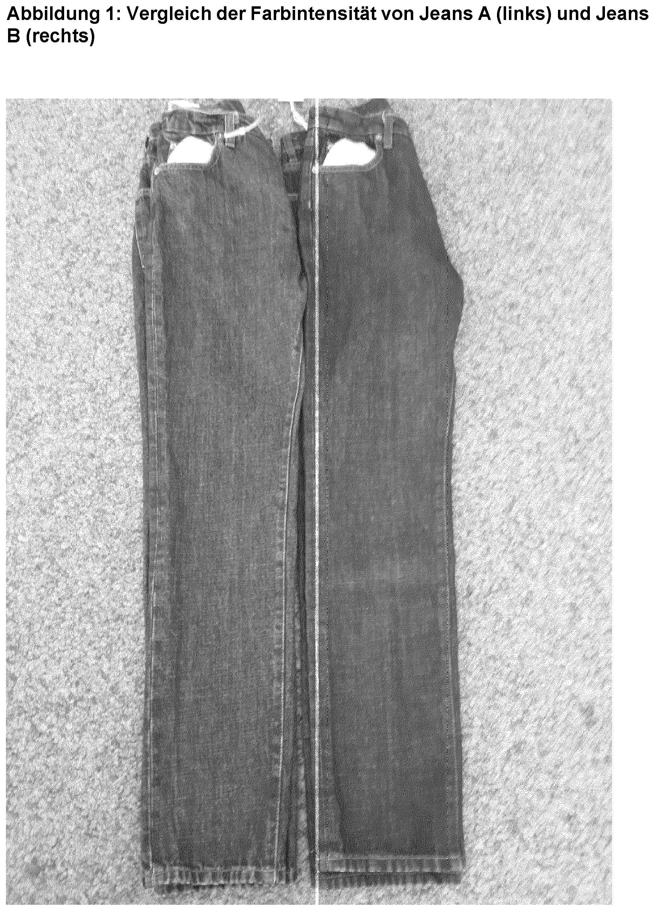

Dabei hat sich gezeigt, dass die Aufhellung durch die erfindungsgemäße Zusammensetzung 3.1 % höher war im Vergleich zur Jeans, die mit der Zusammensetzung des Standes der Technik behandelt wurde (Abbildung 1).

Gleichzeitig fiel das Backstaining an der Hoseninnentasche um 0,52 Punkte geringer aus, d.h. die Hoseninnentasche der Jeans A ist heller als die Hoseninnentasche der Vergleichsjeans Jeans B.

In weiteren Experimenten konnte überdies eine Aufhellung von 6.5 % und mehr beobachtet werden.

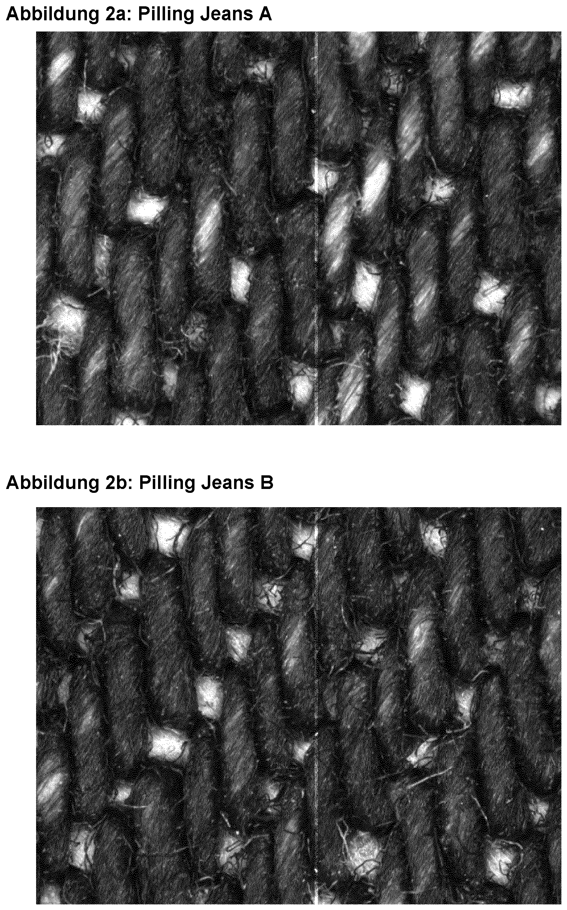

Beim Pilling, d.h. der Entstehung von Faserknötchen, konnte kein Unterschied festgestellt werden. Die erfindungsgemäße Zusammensetzung A (Abbildung 2a) hat das Textil somit nicht mehr strapaziert als die Zusammensetzung B (Abbildung 2b).

Weitere Experimente zeigten sogar weniger lose Faserenden nach der Behandlung mit Zusammensetzung A im Vergleich zur Behandlung mit Zusammensetzung B.

**Beispiel 2** (Messung der Farbintensität)

Die Farbintensität wurde in einem Datacolor 600 Farbmessgerät der Firma Datacolor mit der Software Datacolor TOOLS im Messbereich zwischen 380 und 700 nm bestimmt. Als Standard wurde jeweils eine Jeans mit 100% Farbtiefe als Standard gesetzt. Die Farbmessung erfolgte gemäß DIN 5033-1 und DIN 6176.

**Beispiel 3** (Backstaining)

Um das Backstaining, also die Anfärbung ungefärbter Teile wie die Innentaschen nochmal gesondert zu untersuchen, wurde jeweils eine Jeans mit den Zusammensetzungen A und C-E (siehe Tabelle 1) wie in Beispiel 1 behandelt und die Resultate des "Stone-washings" (Tabelle 3) miteinander verglichen.

Tabelle 3: Backstaining der Jeans A sowie C-E (Standard: Jeans C)

| Ergebnis | Jeans A | Jeans C (Vergleich) Standard | Jeans D (Vergleich) | Jeans E (Vergleich) |
|---|---|---|---|---|
| Backstaining an der Hoseninnentasche Farbintensität [%] | 211,6 | 100 | 163,8 | 205,0 |
| Backstaining an der Hoseninnentasche [DL] | 2,19 | 0 | 0,38 | 1,95 |

Das additive (theoretische) Backstaining aus Jeans D und Jeans E (0,38+1,95 = 2,33 Punkte) ist stärker ausgeprägt als bei Jeans A (2,19 Punkte), was auf einen synergistischen Effekt von Cellulase und ligninhaltigem Material schließen lässt.

**Beispiel 4** (Viskosimetrische Bestimmung der Cellulase-Aktivität)

Der enzymatische Abbau von löslicher Carboxymethylcellulose (CMC) und die damit verbundene Viskositätsänderung kann als Nachweis für die Aktivität von Cellulasen verwendet werden. Im Allgemeinen wird unter einer Enzym-Einheit (Unit) die Menge an Enzym verstanden, um aus einem Substrat ein nmol reduzierenden Zucker wie Glukose in einer Sekunde herzustellen (Measurement of cellulase activities, Pure & Appl.Chem., Vol. 59, No. 2. pp.257-268, 1987).

Um die Cellulase-Aktivität in den vergleichbaren Zusammensetzungen von Beispiel 1 zu überprüfen, wurde eine viskosimetrische Bestimmung vorgenommen.

Die Messungen wurden in einem HAAKE Rotationsviskosimeter VT 500 mit Hilfe der Software Rheowin PR= 2.97 und einem MVDIN-Sensor bei 40°C durchgeführt.

Zuerst wurde eine 2,3%ige CMC-Lösung hergestellt, indem 2,3 % Tylopur C33 P2 der Fa. SE Tylose GmbH&Co.KG in dest. Wasser gelöst wurde. Die Suspension wurde für 1 Stunde gerührt bis die CMC vollständig gelöst war. 40 g der CMC-Lösung wurden dann mit 5,56 ml Acetatpuffer, pH 5.0 versetzt und auf der Heizplatte auf 40°C erwärmt. Die 40°C-warme CMC-Lösung wurde in das Viskosimeter (Einstellung 300 Units / min) transferiert und jeweils 10 min der Viskositätsverlauf aufgezeichnet. Nach Ende der Messung wurde mit Hilfe der Software eine Mittelwertbestimmung der Messdaten durchgeführt (Blindbestimmung).

Zur Messung der Enzymaktivität wurden 40 g CMC-Lösung mit 5 ml Acetatpuffer, pH 5,0 versetzt und auf 40°C erwärmt. Die Prüflösungen wurden mit je 0,05 Gew.-% der Zusammensetzung A und B in Acetatpuffer, pH 5, hergestellt. Nach exakter Temperatureinstellung wurden jeweils 0,3 g neutrale Cellulase (8000 U/g) in Acetatpuffer, pH 5,0 zugegeben und die Lösung exakt 15 Sekunden gerührt. Die Probenlösung wurde anschließend sofort in das Messgefäß des Viskosimeters transferiert. Exakt 30 Sekunden nach der Enzymzugabe wurde die Messung gestartet und der der Verlauf der Viskosität für 10 min bei 40°C aufgezeichnet.

Nach Ende der Messung wurde mit Hilfe der Software eine Regressionsanalyse (Linear var) durchgeführt. Die Enzym-Aktivität ergibt sich aus folgender Gleichung:

$$\text{Aktivität} = F \times B/E$$

E = Enzymmenge im Test (mg)
B = Steigungsfaktor aus Linear var

$$F = \text{CMC-Faktor} = 1 \times 10^8.$$

Die errechnete Aktivität für die Zusammensetzung B betrug 3200 Einheiten (Units), die für die erfindungsgemäße Zusammensetzung A 4900 Einheiten.

Entgegen der erwarteten Aktivitätsverminderung aufgrund der konkurrierenden bzw. inhibierenden Bestandteile von Cellulose und/oder Lignin des ligninhaltigen Materials (hier: Kernschalenmehl), wurde überraschenderweise sogar eine Aktivitätssteigerung festgestellt. Es kann daher davon ausgegangen werden, dass das ligninhaltige Material Enzymaktivatoren enthält, die die Cellulase-Aktivität erhöhen.

**Beispiel 5** (Vergleich mit Standard Jeans C)

Ein Paar fabrikneuer Denim-Jeans (indigo- und schwefelgefärbtes Baumwollgewebe) mit einem Gewicht von ca. 556 g wurden in eine Trommelwaschmaschine des Typs TONELLO G1 Modell 70 L1 Ecofree (Fassungsvermögen 70 l) mit 1670 g Wasser versetzt und überschüssiges Wasser entfernt (abgepumpt).

Zu den feuchten Jeans wurden anschließend je 55 g der Zusammensetzung A-G in trockener Form zugesetzt und bei 20°C für 30 min bei 24 Umin$^{-1}$ mit Umkehr der Rotationsrichtung alle zwei Minuten behandelt.

Die Jeans wurden anschließend für 1 min bei 20°C in jeweils 2780 g Leitungswasser gespült. Das Wasser wurde in einer Schleuder der Marke TONELLO Modell Hydro 12 5 min bei 300 Umin$^{-1}$ und anschließend in einem Trockner "TONELLO G1 Modell TD 25 E" bei 70°C ca. 40 min vollständig entfernt.

Es wurde die Änderung der Helligkeit am jeweils rechten Hosenbein außen (Abrasiv-Effekt) und an der jeweils rechten Innentasche untersucht (Backstaining-Effekt).

In Tabelle 4 ist die Farbintensität sowie das Backstaining der so behandelten Jeans AG zusammengefasst.

Als Standard (100% Farbintensität) wurde Jeans C gewählt, die mit der Zusammensetzung C (kein ligninhaltiges Material und keine Cellulase) behandelt wurde.

Tabelle 4: Ergebnisse der "Stone-Washing"-Behandlung

| Jeans | Farbintensität rechtes Hosenbein unten [%] | Farbintensität rechte Hoseninnentasche [%] (Backstaining) |
|---|---|---|
| A | 88,1 | 228,5 |
| B | 90,1 | 286,4 |
| C | 100 | 100 |
| D | 100 | 216,0 |
| E | 97,1 | 301,3 |
| F | 93,4 | n.b. |
| G | 89,0 | 178 |

Jeans A zeigt eine höhere Aufhellung als Jeans B (Silikat). Jeans A zeigt auch eine höhere Aufhellung gegenüber Jeans D (keine Cellulase) und Jeans E (kein ligninhaltiges Material). Jeans A zeigt auch eine höhere Aufhellung gegenüber der Summe aus Jeans D (keine Cellulase) und Jeans E (kein ligninhaltiges Material). Es wird daher eine synergistische Wirkung von Enzym und ligninhaltigem Material angenommen. Andererseits ist das Backstaining in Jeans A vergleichbar mit dem in Jeans D und reduziert gegenüber Jeans B und E.

In Jeans F (Partikeldurchmesser: 350 $\mu$m) ist die Aufhellung gegenüber Jeans A etwas geringer.

Ferner zeigen die Ergebnisse, dass selbst bei geringen Konzentrationen an Cellulase (Jeans G) sehr gute Ergebnisse bei Aufhellung und sogar ein verbessertes Backstaining im Vergleich zu Jeans A erhalten werden.

**Patentansprüche**

1. Zusammensetzung zur Behandlung von textilen Flächengebilden, enthaltend

(a) mindestens eine Cellulase, insbesondere ausgewählt aus Endo-1,4-$\beta$-Glucanasen, Exo-1,4-$\beta$-Glucanasen und $\beta$-Glucosidasen,
(b) mindestens ein ligninhaltiges Material, das bevorzugt 16-45 Gew.-% Lignin bezogen auf die Gesamtmasse der Komponente (b) enthält,
(c) mindestens einen pH-Regulator, insbesondere ausgewählt aus einem wasserlöslichen anorganischen Salz und/oder wasserlöslichen organischen Salz,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und
(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat.

2. Trockene Zusammensetzung zur Behandlung von textilen Flächengebilden nach Anspruch 1, enthaltend

(a) mindestens eine Cellulase, insbesondere ausgewählt aus Endo-1,4-$\beta$-Glucanasen, Exo-1,4-$\beta$-Glucanasen und $\beta$-Glucosidasen,
(b) mindestens ein ligninhaltiges Material, das bevorzugt 16-45 Gew.-% Lignin bezogen auf die Gesamtmasse der Komponente (b) enthält,
(c) mindestens einen pH-Regulator, insbesondere ausgewählt aus einem wasserlöslichen anorganischen Salz und/oder wasserlöslichen organischen Salz,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und
(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat.

3. Zusammensetzung nach Anspruch 1 oder 2, wobei

der Cellulase-Anteil 0,01-5 Gew.-%, bevorzugt 0,05-2 Gew.-% und am stärksten bevorzugt 0,1-1 Gew.-%, und/oder
der Anteil an ligninhaltigem Material (b) 5-99 Gew.-%, bevorzugt 10-20 Gew.-%, und/oder
der Anteil an pH-Regulator (c) 1-80 Gew.-%, bevorzugt 10-70 Gew.-%, am stärksten bevorzugt 40-70 Gew.-%, und/oder
der Anteil an Tensid (d) 0-40 Gew.-%, bevorzugt 1,5 bis 30 Gew.-%, stärker bevorzugt 5-30 Gew.-%, am stärksten bevorzugt 10-20 Gew.-%, und/oder der Anteil an Anti-Backstaining-Mittel (e) 0-10 Gew.-%, bevorzugt 1-5 Gew.-%, jeweils bezogen auf die Gesamtmasse der Zusammensetzung, beträgt.

4. Zusammensetzung nach einem der Ansprüche 1-3, wobei die Cellulase neutral ist und bevorzugt eine Enzymaktivität von mindestens 300 U/g aufweist, stärker bevorzugt 1000 - 10000 U/g, am meisten bevorzugt 5000 - 9000 U/g.

5. Zusammensetzung nach einem der vorhergehenden Ansprüche, wobei das ligninhaltige Material (b) enthält:

16-45 Gew.-%, bevorzugt 25-45 Gew.-%, Lignin,
30-45 Gew.-%, bevorzugt 34-43 Gew.-%, Cellulose und
20-30 Gew.-%, bevorzugt 22-29 Gew.-%, Hemicellulose,
jeweils bezogen auf die Gesamtmasse der Komponente (b).

6. Zusammensetzung nach einem der vorhergehenden Ansprüche, wobei das ligninhaltige Material (b) aus Nussschalen, insbesondere Walnuss-, Haselnuss-, Mandel-, Kokosnuss-, Pistazien-, Macadamianuss-, Pekannussschalen oder Mischungen davon, ist und/oder aus Kernschalen von Steinobst, insbesondere Oliven, Aprikosen, Pfirsich, Kirsche, und Pflaume, Pinien, Palmfrüchten, Jojoba-Früchten, Kakaobohnen oder Mischungen davon ist.

7. Zusammensetzung nach einem der vorhergehenden Ansprüche, wobei das ligninhaltige Material (b)

einen durchschnittlichen Partikeldurchmesser von 100 bis 250 $\mu$m, bevorzugt von 120 bis 200 $\mu$m und am meisten bevorzugt von 140 bis 170 $\mu$m gemessen nach DIN 66165, und/oder
eine Mohs-Härte von 1,5 bis 5,5, bevorzugt von 2 bis 4, gemessen gemäß DIN EN 101, und/oder
eine Schüttdichte von 300 bis 700 kg/m$^3$, bevorzugt 400 bis 600 kg/m$^3$, gemessen in Anlehnung an DIN ISO EN 60, aufweist.

8. Zusammensetzung nach einem der vorhergehenden Ansprüche, wobei das ligninhaltige Material (b) Petrolether-lösliche Bestandteile von 0,01-3 Gew.-%, bevorzugt 1-2 Gew.-%, bezogen auf die Gesamtmasse der Komponente (b) gemessen in Anlehnung an ISO 734, und/oder ferner Mono- und/oder Polysaccharide, insbesondere Saccharose, Glukose, Xylose, Mannitol und/oder Myoinositol, enthält.

9. Zusammensetzung nach einem der Ansprüche 2-8, wobei die Zusammensetzung einen Wassergehalt von <5 Gew.-%, bevorzugt 0,001-2 Gew.-%, bezogen auf die Gesamtmasse der Zusammensetzung, und/oder eine Schüttdichte von 800 bis 1500 kg/m$^3$, bevorzugt von 1000 bis 1200 kg/m$^3$, aufweist.

10. Kit umfassend mindestens zwei getrennt voneinander vorliegende Einheiten, wobei

die erste Einheit umfasst:

(a) mindestens eine Cellulase, insbesondere ausgewählt aus Endo-1,4-$\beta$-Glucanasen, Exo-1,4-$\beta$-Glucana-sen und $\beta$-Glucosidasen,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und
(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat; und

die zweite Einheit umfasst:

(b) mindestens ein ligninhaltiges Material, das bevorzugt 16-45 Gew.-% Lignin bezogen auf die Gesamtmasse der Komponente (b) enthält,
(c) mindestens einen pH-Regulator, insbesondere ausgewählt aus einem wasserlöslichen anorganischen Salz und/oder wasserlöslichen organischen Salz,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und
(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat;
oder

die erste Einheit umfasst:

(a) mindestens eine Cellulase, insbesondere ausgewählt aus Endo-1,4-β-Glucanasen, Exo-1,4-β-Glucanasen und β-Glucosidasen,
(c) mindestens einen pH-Regulator, insbesondere ausgewählt aus einem wasserlöslichen anorganischen Salz und/oder wasserlöslichen organischen Salz,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und
(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat; und

die zweite Einheit umfasst:

(b) mindestens ein ligninhaltiges Material, das bevorzugt 16-45 Gew.-% Lignin bezogen auf die Gesamtmasse der Komponente (b) enthält,
(d) gegebenenfalls mindestens ein Tensid, insbesondere ein nichtionisches Tensid, bevorzugt polyalkoxylierter Fettalkohol, polyalkoxyliertes Fettamin, polyalkoxylierte Fettsäure, polyalkoxyliertes Fettsäureamid, polyalkoxyliertes Mono-, Di- oder Triglycerid, polyalkoxyliertes Alkylphenol, Fettsäurealkanolamid, Copolymer von Ethylenoxid und Propylenoxid, polyalkoxyliertes Polyol, Ester von Fettsäuren mit polyalkoxylierten Polyolen, Aminoxid oder Amidoaminoxid, und
(e) gegebenenfalls mindestens ein Anti-Backstaining-Mittel, insbesondere ausgewählt aus PEG-Polyester, Polyvinylpyrrolidon, Natriumcarboxymethylcellulose, Hydroxypropylmethylcellulose, Hydroxymethylcellulose, Hydroxyethylcellulose, Polyethylenglycol und polymerem Polycarboxylat.

11. Verwendung einer Zusammensetzung nach einem der Ansprüche 1-9 oder eines Kits nach Anspruch 10 zur Behandlung von textilen Flächengebilden, insbesondere zum Aufhellen von gefärbten textilen Flächengebilden.

12. Verfahren zur Behandlung von textilen Flächengebilden, umfassend die Schritte:

(i) Bereitstellen eines wasserfeuchten textilen Flächengebildes, wobei das textile Flächengebilde bevorzugt einen Wassergehalt von 70-400 Gew.-%, stärker bevorzugt 70-100 Gew.-%, noch stärker bevorzugt 90-100 Gew.-% oder 200-300 Gew.-%, bezogen auf das Trockengewicht des textilen Flächengebildes, gemessen nach DIN 54201, aufweist,
(ii) Zugeben einer Zusammensetzung nach einem der Ansprüche 1-9 oder eines Kits nach Anspruch 10, wobei das Gewichtsverhältnis textiles Flächengebilde: Zusammensetzung bzw. Gesamtheit der Kit-Komponnten bevorzugt 100:0,5-10, stärker bevorzugt 100 : 1-10, noch stärker bevorzugt 100:0,5-5, noch stärker bevorzugt 100:1-5, noch stärker bevorzugt 100 : 2-5 beträgt,

(iii) Bewegen der nach Schritt (ii) erhaltenen Mischung in einem Behälter,

(iv) Waschen des nach Schritt (iii) erhaltenen textilen Flächengebildes und

(v) Isolieren und ggf. Trocknen des nach Schritt (iv) erhaltenen textilen Flächengebildes.

**Claims**

1. A composition for the treatment of textile fabrics, comprising

   (a) at least one cellulase, in particular selected from endo-1,4-β-glucanases, exo-1,4-β-glucanases and β-glucosidases,

   (b) at least one lignin-containing material, which preferably contains 16-45% by weight of lignin based on the total mass of component (b),

   (c) at least one pH regulator, in particular selected from a water-soluble inorganic salt and/or water-soluble organic salt,

   (d) optionally at least one surfactant, in particular a non-ionic surfactant, preferably polyalkoxylated fatty alcohol, polyalkoxylated fatty amine, polyalkoxylated fatty acid, polyalkoxylated fatty acid amide, polyalkoxylated mono-, di- or triglyceride, polyalkoxylated alkylphenol, fatty acid alkanolamide, copolymer of ethylene oxide and propylene oxide, polyalkoxylated polyol, ester of fatty acids with polyalkoxylated polyols, amine oxide or amidoamine oxide, and

   (e) optionally at least one anti-backstaining agent, in particular selected from PEG polyester, polyvinylpyrrolidone, sodium carboxymethylcellulose, hydroxypropylmethylcellulose, hydroxymethylcellulose, hydroxyethylcellulose, polyethylene glycol and polymeric polycarboxylate.

2. The dry composition for the treatment of textile fabrics according to claim 1, comprising

   (a) at least one cellulase, in particular selected from endo-1,4-β-glucanases, exo-1,4-β-glucanases and β-glucosidases,

   (b) at least one lignin-containing material, which preferably contains 16-45% by weight of lignin based on the total mass of component (b),

   (c) at least one pH regulator, in particular selected from a water-soluble inorganic salt and/or water-soluble organic salt,

   (d) optionally at least one surfactant, in particular a non-ionic surfactant, preferably polyalkoxylated fatty alcohol, polyalkoxylated fatty amine, polyalkoxylated fatty acid, polyalkoxylated fatty acid amide, polyalkoxylated mono-, di- or triglyceride, polyalkoxylated alkylphenol, fatty acid alkanolamide, copolymer of ethylene oxide and propylene oxide, polyalkoxylated polyol, ester of fatty acids with polyalkoxylated polyols, amine oxide or amidoamine oxide, and

   (e) optionally at least one anti-backstaining agent, in particular selected from PEG polyester, polyvinylpyrrolidone, sodium carboxymethylcellulose, hydroxypropylmethylcellulose, hydroxymethylcellulose, hydroxyethylcellulose, polyethylene glycol and polymeric polycarboxylate.

3. The composition according to claim 1 or 2, wherein

   the cellulase content is 0.01-5% by weight, preferably 0.05-2% by weight and most preferably 0.1-1% by weight, and/or

   the proportion of lignin-containing material (b) is 5-99% by weight, preferably 10-20% by weight, and/or

   the proportion of pH regulator (c) is 1-80% by weight, preferably 10-70% by weight, most preferably 40-70% by weight, and/or

   the proportion of surfactant (d) is 0-40% by weight, preferably 1.5 to 30% by weight, more preferably 5-30% by weight, most preferably 10-20% by weight, and/or

   the proportion of anti-backstaining agent (e) is 0-10% by weight, preferably 1-5% by weight, in each case based on the total mass of the composition.

4. The composition according to any of claims 1-3, wherein the cellulase is neutral and preferably has an enzyme activity of at least 300 U/g, more preferably 1000 - 10000 U/g, most preferably 5000 - 9000 U/g.

**5.** The composition according to any of the preceding claims, wherein the lignin-containing material (b) comprises:

> 16-45% by weight, preferably 25-45% by weight, lignin,
> 30-45% by weight, preferably 34-43% by weight, cellulose and
> 20-30% by weight, preferably 22-29% by weight, hemicellulose,
> in each case based on the total mass of component (b).

**6.** The composition according to any of the preceding claims, wherein the lignin-containing material (b) is made from nut shells, in particular walnut, hazelnut, almond, coconut, pistachio, macadamia nut, pecan nut shells or mixtures thereof, and/or pits from stone fruit, in particular olive, apricot, peach, cherry, and plum, pine, palm fruit, jojoba fruit, cocoa beans or mixtures thereof.

**7.** The composition according to any of the preceding claims, wherein the lignin-containing material (b)

> has an average particle diameter of 100 to 250 $\mu$m, preferably of 120 to 200 $\mu$m and most preferably of 140 to 170 $\mu$m measured according to DIN 66165, and/or
> has a Mohs hardness of 1.5 to 5.5, preferably 2 to 4, measured according to DIN EN 101, and/or
> has a bulk density of 300 to 700 kg/m$^3$, preferably 400 to 600 kg/m$^3$, measured in accordance with DIN ISO EN 60.

**8.** The composition according to any of the preceding claims, wherein the lignin-containing material (b) contains petroleum ether-soluble constituents of 0.01-3% by weight, preferably 1-2% by weight, based on the total mass of component (b) measured in accordance with ISO 734, and/or further contains mono- and/or polysaccharides, in particular sucrose, glucose, xylose, mannitol and/or myoinositol.

**9.** The composition according to any of claims 2-8, wherein the composition has a water content of <5% by weight, preferably 0.001-2% by weight, based on the total mass of the composition, and/or a bulk density of from 800 to 1500 kg/m$^3$, preferably from 1000 to 1200 kg/m$^3$.

**10.** A Kit comprising at least two separately present units, wherein

> the first unit comprises:
>
> > (a) at least one cellulase, in particular selected from endo-1,4-$\beta$-glucanases, exo-1,4-$\beta$-glucanases and $\beta$-glucosidases,
> > (d) optionally at least one surfactant, in particular a nonionic surfactant, preferably polyalkoxylated fatty alcohol, polyalkoxylated fatty amine, polyalkoxylated fatty acid, polyalkoxylated fatty acid amide, polyalkoxylated mono-, di- or triglyceride, polyalkoxylated alkylphenol, fatty acid alkanolamide, copolymer of ethylene oxide and propylene oxide, polyalkoxylated polyol, ester of fatty acids with polyalkoxylated polyols, amine oxide or amidoamine oxide, and
> > (e) optionally at least one anti-backstaining agent, in particular selected from PEG-polyester, polyvinylpyrrolidone, sodium carboxymethylcellulose, hydroxypropylmethylcellulose, hydroxymethylcellulose, hydroxyethylcellulose, polyethylene glycol and polymeric polycarboxylate; and
>
> the second unit comprises:
>
> > (b) at least one lignin-containing material, which preferably contains 16-45% by weight of lignin based on the total mass of component (b),
> > (c) at least one pH regulator, in particular selected from a water-soluble inorganic salt and/or water-soluble organic salt,
> > (d) optionally at least one surfactant, in particular a non-ionic surfactant, preferably polyalkoxylated fatty alcohol, polyalkoxylated fatty amine, polyalkoxylated fatty acid, polyalkoxylated fatty acid amide, polyalkoxylated mono-, di- or triglyceride, polyalkoxylated alkylphenol, fatty acid alkanolamide, copolymer of ethylene oxide and propylene oxide, polyalkoxylated polyol, ester of fatty acids with polyalkoxylated polyols, amine oxide or amidoamine oxide, and
> > (e) optionally at least one anti-backstaining agent, in particular selected from PEG polyester, polyvinylpyrrolidone, sodium carboxymethylcellulose, hydroxypropylmethylcellulose, hydroxymethylcellulose, hydroxyethylcellulose, polyethylene glycol and polymeric polycarboxylate;
> > or

the first unit comprises:

(a) at least one cellulase, in particular selected from endo-1,4-β-glucanases, exo-1,4-β-glucanases and β-glucosidases,
(c) at least one pH regulator, in particular selected from a water-soluble inorganic salt and/or water-soluble organic salt,
(d) optionally at least one surfactant, in particular a non-ionic surfactant, preferably polyalkoxylated fatty alcohol, polyalkoxylated fatty amine, polyalkoxylated fatty acid, polyalkoxylated fatty acid amide, polyalkoxylated mono-, di- or triglyceride, polyalkoxylated alkylphenol, fatty acid alkanolamide, copolymer of ethylene oxide and propylene oxide, polyalkoxylated polyol, ester of fatty acids with polyalkoxylated polyols, amine oxide or amidoamine oxide, and
(e) optionally at least one anti-backstaining agent, in particular selected from PEG-polyester, polyvinylpyrrolidone, sodium carboxymethylcellulose, hydroxypropylmethylcellulose, hydroxymethylcellulose, hydroxyethylcellulose, polyethylene glycol and polymeric polycarboxylate; and

the second unit comprises:

(b) at least one lignin-containing material, which preferably contains 16-45% by weight of lignin based on the total mass of component (b)
(d) optionally at least one surfactant, in particular a non-ionic surfactant, preferably polyalkoxylated fatty alcohol, polyalkoxylated fatty amine, polyalkoxylated fatty acid, polyalkoxylated fatty acid amide, polyalkoxylated mono-, di- or triglyceride, polyalkoxylated alkylphenol, fatty acid alkanolamide, copolymer of ethylene oxide and propylene oxide, polyalkoxylated polyol, ester of fatty acids with polyalkoxylated polyols, amine oxide or amidoamine oxide, and
(e) optionally at least one anti-backstaining agent, in particular selected from PEG polyester, polyvinylpyrrolidone, sodium carboxymethylcellulose, hydroxypropylmethylcellulose, hydroxymethylcellulose, hydroxyethylcellulose, polyethylene glycol and polymeric polycarboxylate.

11. A use of a composition according to any of claims 1-9 or of a kit according to claim 10 for the treatment of textile fabrics, in particular for brightening dyed textile fabrics.

12. A process for the treatment of textile fabrics, comprising the steps of:

(i) providing a water-moist textile fabric, the textile fabric preferably having a water content of 70-400% by weight, more preferably 70-100% by weight, even more preferably 90-100% by weight or 200-300% by weight, based on the dry weight of the textile fabric, measured according to DIN 54201,
(ii) adding a composition according to any of claims 1-9 or a kit according to claim 10, wherein the weight ratio of textile fabric: composition or total of kit components is preferably 100:0.5-10, more preferably 100:1-10, even more preferably 100:0.5-5, even more preferably 100:1-5, even more preferably 100:2-5,
(iii) moving the mixture obtained after step (ii) in a container,
(iv) washing the textile fabric obtained after step (iii) and
(v) isolating and, optionally drying the textile fabric obtained after step (iv).

**Revendications**

1. Composition pour le traitement de structures textiles planes, contenant

(a) au moins une cellulase, en particulier choisie parmi les endo-1,4-β-glucanases, les exo-1,4-β-glucanases et les β-glucosidases,
(b) au moins une matière contenant de la lignine, qui contient de préférence 16 à 45 % en poids de lignine par rapport à la masse totale du composant (b),
(c) au moins un régulateur de pH, en particulier choisi parmi un sel inorganique hydrosoluble et/ou un sel organique hydrosoluble,
(d) le cas échéant, au moins un tensioactif, en particulier un tensioactif non ionique, de préférence un alcool gras polyalcoxylé, une amine grasse polyalcoxylée, un acide gras polyalcoxylé, un amide d'acide gras polyalcoxylé, un mono-, di- ou triglycéride polyalcoxylé, un alkylphénol polyalcoxylé, un alcanolamide d'acide gras, un copolymère d'oxyde d'éthylène et d'oxyde de propylène, un polyol polyalcoxylé, un ester d'acides gras avec des polyols polyalcoxylés, un oxyde d'amine ou un oxyde d'amidoamine, et

(e) le cas échéant, au moins un agent anti-rétrocoloration, en particulier choisi parmi le polyester PEG, la polyvinylpyrrolidone, la carboxyméthylcellulose de sodium, l'hydroxypropylméthylcellulose, l'hydroxyméthylcellulose, l'hydroxyéthylcellulose, le polyéthylèneglycol et le polycarboxylate polymère.

2. Composition sèche pour le traitement de structures textiles planes selon la revendication 1, contenant

(a) au moins une cellulase, en particulier choisie parmi les endo-1,4-β-glucanases, les exo-1,4-β-glucanases et les β-glucosidases,
(b) au moins une matière contenant de la lignine, qui contient de préférence 16 à 45 % en poids de lignine par rapport à la masse totale du composant (b),
(c) au moins un régulateur de pH, en particulier choisi parmi un sel inorganique hydrosoluble et/ou un sel organique hydrosoluble,
(d) le cas échéant, au moins un tensioactif, en particulier un tensioactif non ionique, de préférence un alcool gras polyalcoxylé, une amine grasse polyalcoxylée, un acide gras polyalcoxylé, un amide d'acide gras polyalcoxylé, un mono-, di- ou triglycéride polyalcoxylé, un alkylphénol polyalcoxylé, un alcanolamide d'acide gras, un copolymère d'oxyde d'éthylène et d'oxyde de propylène, un polyol polyalcoxylé, un ester d'acides gras avec des polyols polyalcoxylés, un oxyde d'amine ou un oxyde d'amidoamine, et
(e) le cas échéant, au moins un agent anti-rétrocoloration, en particulier choisi parmi le polyester PEG, la polyvinylpyrrolidone, la carboxyméthylcellulose de sodium, l'hydroxypropylméthylcellulose, l'hydroxyméthylcellulose, l'hydroxyéthylcellulose, le polyéthylèneglycol et le polycarboxylate polymère.

3. Composition selon la revendication 1 ou 2, dans laquelle

la teneur en cellulase est de 0,01 à 5 % en poids, de préférence de 0,05 à 2 % en poids et, de manière particulièrement préférée, de 0,1 à 1 % en poids, et/ou
la teneur en matière contenant de la lignine (b) est de 5 à 99 % en poids, de préférence de 10 à 20 % en poids, et/ou
la teneur en régulateur de pH (c) est de 1 à 80 % en poids, de préférence de 10 à 70 % en poids, de manière particulièrement préférée de 40 à 70 % en poids, et/ou
la teneur en tensioactif (d) est de 0 à 40 % en poids, de préférence de 1,5 à 30 % en poids, de préférence encore de 5 à 30 % en poids, de manière particulièrement préférée de 10 à 20 % en poids, et/ou
la teneur en agent anti-rétrocoloration (e) est de 0 à 10 % en poids, de préférence de 1 à 5 % en poids, par rapport à la masse totale de la composition.

4. Composition selon l'une des revendications 1 à 3, dans laquelle la cellulase est neutre et présente de préférence une activité enzymatique d'au moins 300 U/g, de préférence de 1 000 à 10 000 U/g, de manière particulièrement préférée de 5 000 à 9 000 U/g.

5. Composition selon l'une des revendications précédentes, dans laquelle la matière contenant de la lignine (b) contient :

16 à 45 % en poids, de préférence 25 à 45 % en poids, de lignine,
30 à 45 % en poids, de préférence 34 à 43 % en poids, de cellulose et
20 à 30 % en poids, de préférence 22 à 29 % en poids, d'hémicellulose,
par rapport à la masse totale du composant (b).

6. Composition selon l'une des revendications précédentes, dans laquelle la matière contenant de la lignine (b) est constituée de coques de fruits à coque, en particulier de coques de noix, de noisettes, d'amandes, de noix de coco, de pistaches, de noix de macadamia, de noix de pécan ou de mélanges de celles-ci, et/ou de noyaux de fruits à noyau, en particulier d'olives, d'abricots, de pêches, de cerises et de prunes, de pignons, de fruits de palmier, de fruits de jojoba, de fèves de cacao ou de mélanges de ceux-ci.

7. Composition selon l'une des revendications précédentes,

dans laquelle la matière contenant de la lignine (b)
a un diamètre moyen de particules de 100 à 250 $\mu$m, de préférence de 120 à 200 $\mu$m et de manière particulièrement préférée de 140 à 170 $\mu$m, mesuré selon la norme DIN 66165, et/ou
une dureté Mohs de 1,5 à 5,5, de préférence de 2 à 4, mesurée selon la norme DIN EN 101, et/ou
une masse volumique apparente de 300 à 700 kg/m$^3$, de préférence de 400 à 600 kg/m$^3$, mesurée selon la norme DIN ISO EN 60.

8. Composition selon l'une des revendications précédentes,
dans laquelle la matière contenant de la lignine (b) contient des composants solubles dans l'éther de pétrole de 0,01 à 3 % en poids, de préférence de 1 à 2 % en poids, par rapport à la masse totale du composant (b), mesurés selon la norme ISO 734, et/ou en outre des mono- et/ou polysaccharides, en particulier du saccharose, du glucose, du xylose, du mannitol et/ou du myo-inositol.

9. Composition selon l'une des revendications 2 à 8,
dans laquelle la composition présente une teneur en eau < 5 % en poids, de préférence de 0,001 à 2 % en poids, par rapport à la masse totale de la composition, et/ou une masse volumique apparente de 800 à 1 500 kg/m$^3$, de préférence de 1 000 à 1 200 kg/m$^3$.

10. Kit comprenant au moins deux unités séparées l'une de l'autre, la première unité contenant :

(a) au moins une cellulase, en particulier choisie parmi les endo-1,4-β-glucanases, les exo-1,4-β-glucanases et les β-glucosidases,
(d) le cas échéant, au moins un tensioactif, en particulier un tensioactif non ionique, de préférence un alcool gras polyalcoxylé, une amine grasse polyalcoxylée, un acide gras polyalcoxylé, un amide d'acide gras polyalcoxylé, un mono-, di- ou triglycéride polyalcoxylé, un alkylphénol polyalcoxylé, un alcanolamide d'acide gras, un copolymère d'oxyde d'éthylène et d'oxyde de propylène, un polyol polyalcoxylé, un ester d'acides gras avec des polyols polyalcoxylés, un oxyde d'amine ou un oxyde d'amidoamine, et
(e) le cas échéant, au moins un agent anti-rétrocoloration, en particulier choisi parmi le polyester PEG, la polyvinylpyrrolidone, la carboxyméthylcellulose de sodium, l'hydroxypropylméthyl-cellulose, l'hydroxyméthylcellulose, l'hydroxyéthylcellulose, le polyéthylèneglycol et le polycarboxylate polymère ; et
la deuxième unité contient :

(b) au moins une matière contenant de la lignine, qui contient de préférence 16 à 45 % en poids de lignine par rapport à la masse totale du composant (b),
(c) au moins un régulateur de pH, en particulier choisi parmi un sel inorganique hydrosoluble et/ou un sel organique hydrosoluble,
(d) le cas échéant, au moins un tensioactif, en particulier un tensioactif non ionique, de préférence un alcool gras polyalcoxylé, une amine grasse polyalcoxylée, un acide gras polyalcoxylé, un amide d'acide gras polyalcoxylé, un mono-, di- ou triglycéride polyalcoxylé, un alkylphénol polyalcoxylé, un alcanolamide d'acide gras, un copolymère d'oxyde d'éthylène et d'oxyde de propylène, un polyol polyalcoxylé, un ester d'acides gras avec des polyols polyalcoxylés, un oxyde d'amine ou un oxyde d'amidoamine, et
(e) le cas échéant, au moins un agent anti-rétrocoloration, en particulier choisi parmi le polyester PEG, la polyvinylpyrrolidone, la carboxyméthylcellulose de sodium, l'hydroxypropylméthyl-cellulose, l'hydroxyméthylcellulose, l'hydroxyéthylcellulose, le polyéthylèneglycol et le polycarboxylate polymère ;
ou

la première unité contient :

(a) au moins une cellulase, en particulier choisie parmi les endo-1,4-β-glucanases, les exo-1,4-β-glucanases et les β-glucosidases,
(c) au moins un régulateur de pH, en particulier choisi parmi un sel inorganique hydrosoluble et/ou un sel organique hydrosoluble,
(d) le cas échéant, au moins un tensioactif, en particulier un tensioactif non ionique, de préférence un alcool gras polyalcoxylé, une amine grasse polyalcoxylée, un acide gras polyalcoxylé, un amide d'acide gras polyalcoxylé, un mono-, di- ou triglycéride polyalcoxylé, un alkylphénol polyalcoxylé, un alcanolamide d'acide gras, une copolymère d'oxyde d'éthylène et d'oxyde de propylène, un polyol polyalcoxylé, un ester d'acides gras avec des polyols polyalcoxylés, un oxyde d'amine ou un oxyde d'amidoamine, et
(e) le cas échéant, au moins un agent anti-rétrocoloration, en particulier choisi parmi le polyester PEG, la polyvinylpyrrolidone, la carboxyméthylcellulose de sodium, l'hydroxypropylméthyl-cellulose, l'hydroxyméthylcellulose, l'hydroxyéthylcellulose, le polyéthylèneglycol et le polycarboxylate polymère ; et

la deuxième unité contient :

(b) au moins une matière contenant de la lignine, qui contient de préférence 16 à 45 % en poids de lignine par rapport à la masse totale du composant (b),

(d) le cas échéant, au moins un tensioactif, en particulier un tensioactif non ionique, de préférence un alcool gras polyalcoxylé, une amine grasse polyalcoxylée, un acide gras polyalcoxylé, un amide d'acide gras polyalcoxylé, un mono-, di- ou triglycéride polyalcoxylé, un alkylphénol polyalcoxylé, un alcanolamide d'acide gras, un copolymère d'oxyde d'éthylène et d'oxyde de propylène, un polyol polyalcoxylé, un ester d'acides gras avec des polyols polyalcoxylés, un oxyde d'amine ou un oxyde d'amidoamine, et

(e) le cas échéant, au moins un agent anti-rétrocoloration, en particulier choisi parmi le polyester PEG, la polyvinylpyrrolidone, la carboxyméthylcellulose de sodium, l'hydroxypropylméthyl-cellulose, l'hydroxyméthylcellulose, l'hydroxyéthylcellulose, le polyéthylèneglycol et le polycarboxylate polymère.

11. Utilisation d'une composition selon l'une des revendications 1 à 9 ou d'un kit selon la revendication 10 pour le traitement de structures textiles planes, en particulier pour éclaircir des structures textiles planes teintes.

12. Procédé de traitement de structures textiles planes, comprenant les étapes consistant à :

(i) fournir une structure textile plane, mouillée à l'eau, la structure textile plane ayant de préférence une teneur en eau de 70 à 400 % en poids, de préférence encore de 70 à 100 % en poids, de préférence encore de 90 à 100 % en poids ou de 200 à 300 % en poids % par rapport au poids sec de la structure textile plane, mesurée selon la norme DIN 54201,

(ii) ajouter une composition selon l'une des revendications 1 à 9 ou un kit selon la revendication 10, le rapport pondéral entre la structure textile plane et la composition ou l'ensemble des composants du kit étant de préférence de 100 : 0,5 à 10, de préférence encore de 100 : 1 à 10, de préférence encore de 100 : 0,5 à 5, de préférence encore de 100 : 1 à 5, de préférence encore de 100 : 2 à 5,

(iii) agiter le mélange, obtenu après l'étape (ii), dans un récipient,

(iv) laver la structure textile plane, obtenue après l'étape (iii), et

(v) isoler et, le cas échéant, sécher la structure textile plane, obtenue après l'étape (iv).

**Abbildung 1: Vergleich der Farbintensität von Jeans A (links) und Jeans B (rechts)**

## Abbildung 2a: Pilling Jeans A

## Abbildung 2b: Pilling Jeans B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9007569 A1
- DE 19643036 A1
- EP 2142698 A1
- CN 1762910 A
- WO 2022106072 A1
- DE 102005049908 A1

**In der Beschreibung aufgeführte Nicht-Patentliteratur**

- **PAN XJ**. Role of functional groups in lignin inhibition of enzymatic hydrolysis of cellulose to glucose. *J. Biobased Mater. Bioenergy*, 2008, vol. 2, 25-32
- *CHEMICAL ABSTRACTS*, 9012-54-8
- **W. C. GRIFFIN**. Classification of surface active agents by HLB. *J. Soc. Cosmet. Chem*, 1949, vol. 1, 311-326
- *Measurement of cellulase activities, Pure & Appl.Chem.*, 1987, vol. 59 (2), 257-268